# EUROPÄISCHE PATENTSCHRIFT

(11) **EP 1 371 979 B1**
(45) Veröffentlichungstag und Bekanntmachung des Hinweises auf die Patenterteilung: **07.12.2005**
(21) Anmeldenummer: 03013039.7
(22) Anmeldetag: 10.06.2003
(51) Int. Cl.: G01N 33/36, G01N 22/00, G01N 22/04

(54) **Mikrowellenvorrichtung zur Qualitätsprüfung von strangförmigen Materialien**
Microwave device for quality tests on strand like materials
Dispositif à micro-ondes pour tester la qualité des matériaux en forme de cordon

(30) Priorität: 12.06.2002 EP 02013000
(43) Veröffentlichungstag der Anmeldung: 17.12.2003
(73) Patentinhaber: TEWS ELEKTRONIK Dipl.-Ing. Manfred Tews, 22459 Hamburg (DE)
(72) Erfinder: Herrmann, Rainer, 20253 Hamburg (DE); Tews, Manfred, 22459 Hamburg (DE); Schlemm, Udo, 20357 Hamburg (DE)
(74) Vertreter: Glawe, Delfs, Moll

(56) Entgegenhaltungen:
- EP-A- 0 266 611
- EP-A- 0 889 321
- WO-A-00/12974
- WO-A-03/050530
- DE-A- 4 342 288
- PATENT ABSTRACTS OF JAPAN vol. 013, no. 001 (P-808), 6. Januar 1989 (1989-01-06) & JP 63 210757 A (NIPPON GLASS FIBER CO LTD), 1. September 1988 (1988-09-01)

## Beschreibung

Die Erfindung betrifft eine Vorrichtung zur Qualitätsprüfung von strangförmigen Materialien in Form von Garnen, Vorgarnen, Faserbändern und dergleichen mit einer Messeinrichtung, die einen Mikrowellengenerator, einen Mikrowellenresonator und Einrichtungen zum Bestimmen der Verschiebung der Resonanzfrequenz und der Verbreiterung der Resonanzkurve durch das strangförmige Material aufweist, und mit mechanischen Einrichtungen zum Transport der strangförmigen Materialien durch das Messvolumen des Mikrowellenresonators.

Für die Qualitätsprüfung von Garnen, Vorgarnen und Faserbändern ist eine Detektion von Qualitätsschwankungen, insbesondere von Masseschwankungen, d. h. eine Überprüfung ihrer Homogenität notwendig. Es müssen dann Fehler bezüglich Masseänderung erfasst werden. Anhand der Häufigkeit und Intensität der Fehler kann eine Qualitätsbewertung des Messgutes erfolgen. Diese Qualitätsbewertung soll vorzugsweise, aber nicht ausschließlich mit einem Labormessplatz bewirkt werden. Die Messung soll unabhängig von Parametern wie Materialfeuchte, Materialfarbe, Materialsorte (z. B. Anbaugebiet von Baumwolle) erfolgen.

Es sind verschiedene Vorrichtungen zur Qualitätsprüfung von strangförmigen Materialien bekannt, die allerdings Nachteile aufweisen. Die Dicke von insbesondere Faserbändern kann mechanisch gemessen werden, indem das Faserband durch ein Paar von Walzen hindurchgeführt wird, von denen wenigstens eine beweglich ist (DE 298 23 928 U1). Durch ein Tastelement kann dann die durch Dickenänderung bewirkte Auslenkung einer der Walzen bestimmt werden. Dieses Verfahren mag für Faserbänder einer konstanten Breite nutzbar sein. Für verhältnismäßig dünne Garne ist es nicht geeignet, da Dickenänderungen in Axialrichtung nur dann erfaßt werden können, wenn wie beim Stand der Technik die Walzen als Nut- und Federwalzen ausgebildet sind.

Es ist auch bekannt, Messungen der Masse mit einer aus Lichtsender und Lichtempfänger bestehenden Anordnung vorzunehmen (DE 29 12 558 C2). Hiermit können aber nur optische Eigenschaften infolge oberflächlicher Reflektionen gemessen werden, die dann die Masse bzw. Masse pro Länge des Materials nicht wiedergeben. Verfälschungen können z. B. bei Schwankungen der Farbe des zu messenden Strangs auftreten. Dieses Verfahren des Standes der Technik ist auch in erster Linie nur für Faserbänder vorgesehen.

Dies gilt auch für ein Verfahren, bei dem Schall durch das Material gesendet wird und aus der Änderung der Dämpfung auf Änderungen der Materialeigenschaften geschlossen wird (DE 32 37 357 C2). Eine eindeutige Bestimmung der Masse ist dadurch nicht möglich, da die Dämpfung auch durch andere Eigenschaften, z. B. die Feuchte des Materials beeinflußt werden kann. Außerdem ist dieses Material für dünnere Garne kaum zu verwenden, da nicht der gesamte Schall durch das Garn hindurchgelenkt werden kann.

Weiter ist es bekannt, das strangförmige Material durch zwei Platten eines Kondensators hindurchlaufen zu lassen (DE-OS 20 41 044). Solche kapazitiven Meßverfahren haben den Nachteil, daß die Signale sehr empfindlich auf Änderungen der Feuchte wegen der hohen Dielektrizitätskonstante von Wasser reagieren. Es kann dadurch der Einfluß von Feuchtevariationen nicht kompensiert werden. Das Material muß daher vor der kapazitiven Messung auf einen genau definierten und bekannten Feuchtewert stabilisiert werden, was ohne weiteres ein bis zwei Tage auch bei gleichmäßigen Temperaturen und sonstigen konstanten Bedingungen erfordern kann. Eine schnelle Messung der Qualität ist damit nicht möglich. Die Feuchtefluktuationen sind dabei besonders störend, je produktionsnaher das Textilmaterial in der Teststation untersucht wird, wenn also nicht für eine genügende Homogenisierung des Materials gesorgt werden kann. Ein präzises Abbild der Qualität des aktuellen Produkts, das frei von Feuchtefluktuationen ist, ergibt sich erst nach einer entsprechend langen Lagerdauer unter entsprechenden Bedingungen. Aber auch dann setzen Feuchte-Inhomogenitäten aufgrund variabler Porenstrukturen, wie sie in allen Produkten auftreten, der Meßpräzision eine deutliche Grenze.

Bei einem Verfahren der eingangs genannten Art (WO-00/12974) wird die Dichte des faserförmigen Strangmaterials bestimmt, d. h. es wird die Masse pro Volumeneinheit gemessen. Ändert sich das Volumen des Materials, das jeweils gemessen wird, ist diese Dichtebestimmung nur möglich, wenn das Volumen der Probe im Meßfeld jeweils bekannt ist. Auch muß der Ort des gemessenen Materialvolumens durch mechanische Probenführung genau konstant gehalten werden, da man verfälschte Signale erhalten wird, wenn der Materialstrang in Bereiche geringerer Mikrowellenstärken abwandert. Ändert sich das Querschnittsvolumen der Probe, muss dies zur Dichtemessung entweder durch eine parallele Volumenmessung erfasst oder durch Neukalibration berücksichtigt werden. Die Dichte ist auch in vielen Fällen von untergeordneter Bedeutung, wenn das Material ohnehin später komprimiert, also auf eine höhere Dichte gebracht wird. Die Dichte ist daher für die Qualitätsprüfung weniger geeignet.

Es ist bekannt, die Feuchte und Dichte von strangförmigen Materialien in einem flachen zylindrischen Resonator zu messen, wobei das Material durch eine Durchgengsbohrung hindurchgeführt wird (EP 0 889 321 A1) . Es ist weiter vorgeschlagen worden, Mikrowellen zum Bestimmen der Masse von Strängen in der Spinnereiindustrie zu verwenden (nachveröffentlichte WO 03/050530 A2) .

Die Aufgabe der Erfindung besteht in der Schaffung einer Vorrichtung der eingangs genannten Art, mit der schnell, zuverlässig und besser die Qualität von strangförmigen Materialien bestimmt werden kann.

Die erfindungsgemäße Lösung besteht darin, dass das Mikrowellenfeld im Messvolumen homogen ist und die Vorrichtung zum Messen der Masse pro Länge und der Feuchte der strangförmigen Materialien ausgebildet ist und dass der Mikrowellenresonator ein oder mehrere auf seiner Längsacke angeordnete metallische Elemente zur Konzentration des Mikrowellenfeldes aufweist.

Es wird also im Gegensatz zum Stand der Technik nicht die Dichte der strangförmigen Materialien gemessen, sondern die Masse pro Länge. Dieser Wert ist wesentlich für die weitere Verarbeitung, damit in nachfolgenden Maschinen immer vergleichbare bzw. bekannte Materialmengen pro Zeiteinheit zugeführt werden. Die pro Zeiteinheit zugeführte Materialmenge ergibt sich dabei aus der bestimmten Masse pro Länge und der Zuführungsgeschwindigkeit.

Damit die Masse pro Länge genau bestimmt werden kann, ist vorgesehen, daß das Mikrowellenmeßfeld über das Probenvolumen im wesentlichen homogen ist. Es spielt daher keine Rolle, wenn das Material in seinen Querschnittsabmessungen Änderungen erfährt oder aber sich etwas aus der Mitte des Meßfeldes herausbewegt. Das Mikrowellensignal wird sich in diesem Falle im Gegensatz zum Verfahren des Standes der Technik nicht ändern, selbst wenn das Material um einen Faktor 10 komprimiert wird.

Durch die besondere Gestaltung des Mikrowellen-Resonatorfeldes mit dafür besonders an die Produktgeometrie angepaßten Resonatoren ist bei der erfindungsgemäßen Vorrichtung das Volumen, das die strangförmigen Materialien beim Durchlaufen des Meßvolumens einnehmen, für das Meßsignal völlig irrelevant, solange sie sich innerhalb gewisser Grenzen bewegten, die für den speziellen Resonator und das spezielle Produkt gültig sind. Wird also das faser- oder strangförmige Material innerhalb dieser Meßvolumengrenzen beim Durchlaufen des Resonators komprimiert, so bleibt das von der Auswerteschaltung erzeugte Massesignal konstant, obwohl sich die Strangdichte um ein Vielfaches ändert. Dies ist auch einer der Gründe, warum bei einer vorteilhaften Ausführungsform die Umschaltung auf mehrere Resonatoren vorgesehen ist. Je nach dem erforderlichen Volumen, das das zu messende Material einnimmt, wird zur Massemessung ein spezieller Resonator verwendet, der im Querschnitt über die Materialprobe ein homogenes elektrisches Magnetfeld aufweist, so daß eine Masseveränderung an einer beliebigen Stelle des Querschnitts zur gleichen Vergrößerung des Massesignals führt. Im anderen Falle sehr dünner Fäden ist es möglich, die nötige Querhomogenität des Meßfeldes auf einen sehr kleinen Querschnittsbereich zu begrenzen, so daß in diesem Falle eine Feldfokussierung auf den Fadenbereich möglich ist, wodurch ein normalerweise sehr kleiner Meßeffekt des dünnen Fadens verstärkt werden kann. Die geforderte Querhomogenität des Meßfeldes für die Massemessung ermöglicht somit auch eine Konstanz des Massesignals, selbst wenn sich die zu messende Probe quer zur Strangrichtung im Bereich des erlaubten Meßfeldes bewegt, wodurch die Meßpräzision der Massemessung deutlich gesteigert wird. Da das Meßfeld in Strangrichtung durch die Resonatorgeometrie definiert ist, wird durch die Bestimmung der Masse pro Länge des strangförmigen Materials die gesamte Masse im Querschnitt der Probe erfaßt, unabhängig von ihrer Verteilung oder Positionierung im Meßfeld des Resonators. Es wird dabei durch die erfindungsgemäße Vorrichtung nicht der Massefluß (Masse pro Zeiteinheit oder Produkt aus Masse und Stranggeschwindigkeit) erfaßt, sondern unabhängig von der Stranggeschwindigkeit die im Meßfeld anwesende Masse.

Bestimmen der Verschiebung der Resonanzfrequenz und der Verbreiterung der Resonanzkurve" bedeutet dabei nicht, daß diese Bestimmung tatsächlich und direkt durch schrittweises Abfahren der Resonanzkurve vorgenommen wird. Diese Bestimmung kann auch indirekt durch Messung an einigen wenigen Punkten der Resonanzkurve vorgenommen werden, wobei dann gewisse Annahmen über die Form dieser Resonanzkurve gemacht werden.

Die Messung wird mit Mikrowellen vorgenommen, wobei im Resonator Elemente zur Konzentration des Mikrowellenfeldes vorgesehen sind. Mit der Mikrowellentechnik steht ein genaues Verfahren zur Verfügung, um Qualitätsparameter, insbesondere die Masse unabhängig von der Feuchtigkeit zu bestimmen (WO 91/12518). Die Mikrowellenmeßtechnik erlaubt es dabei auch, mit sehr hohen Geschwindigkeiten des strangförmigen Materials zu arbeiten, da keine mechanisch bewegten Teile vorgesehen sind, die auf Dickeänderungen reagieren müssen. Vielmehr erfolgt die gesamte Erfassung und Auswertung elektronisch.

Die Messungen können mit Frequenzen von 300 MHz bis 30 GHz durchgeführt werden. Insbesondere haben sich aber Frequenzen zwischen 1 GHz bis 15 GHz als besonders geeignet erwiesen.

Um genaue Messungen zu ermöglichen, sollte durch das durchlaufende strangförmige Material das Feld des Resonators möglichst stark beeinflußt werden. Das Feld des Resonators sollte also weitgehend auf den Raumbereich konzentriert sein, den das strangförmige Material durchläuft. Während man für breite Faserbänder verhältnismäßig große Resonatoren benötigt, durch die das Material hindurchlaufen muß, wird man für dünnere Garne oder Vorgarne kleinere Resonatoren verwenden. Die unterschiedlichen Resonatoren werden dabei über Umschalter mit der Auswertungsschaltung verbunden, so daß immer derjenige Resonator mit der Auswertungsschaltung verbunden ist, durch den gerade das zu messende Material hindurchläuft. Andererseits oder zusätzlich können die Mikrowellenresonatoren Elemente zum Verändern der Mikrowellenfeldkonfiguration aufweisen, damit auch in einem größeren Resonator, durch den ein Material mit geringeren Ausmessungen hindurchläuft, die Mikrowellenstärke auf das Material konzentriert werden kann.

Die Vorrichtung ist zweckmäßigerweise als Labormeßplatz ausgebildet, damit die strangförmigen Materialien vor der Verarbeitung überprüft werden können.

Zusätzlich zu den bereits erwähnten Vorteilen, insbesondere gegenüber dem kapazitiven Verfahren, hat die Mikrowellentechnik den Vorteil, daß die Vorrichtung unmittelbar nach dem Einschalten in Betrieb genommen werden kann. Die Mikrowellengeräte benötigen nicht wie bei den empfindlichen kapazitiven Verfahren eine Warmlaufphase.

Mit der erfindungsgemäßen Vorrichtung kann nicht nur die Feuchte masseunabhängig gemessen werden. Es ist vielmehr insbesondere eine genaue Massemessung möglich, bei der der Einfluß der Feuchte automatisch kompensiert wird, also feuchteunabhängige Massemessungen möglich sind. Letzteres ist von besonderer Bedeutung, da eine Variation der Materialfeuchtigkeit sich durch die große Dielektrizitätskonstante des Wassers empfindlich als Störung einer exakten Massemessung erweist. Durch die automatische Kompensation des Mikrowellen-Massemeßwertes mit Hilfe des Mikrowellen-Feuchtemeßwertes kann diese Fehlerquelle ausgeschieden werden. Die mit Hilfe der Mikrowellenmethode gemessene Masse entspricht so der tatsächlichen Masse des Produkts. Führt man zusätzlich eine Feuchtekalibration auf Basis eines der gängigen Referenzverfahren durch (die innerhalb eines Materialtyps weitgehend sortenunabhängig ist), so ermöglicht das Mikrowellenverfahren auch die Messung der Trockenmaterialmasse oder der Masse bei einer frei definierbaren Zielfeuchte.

Wenn hier von "Masse" die Rede ist, so ist damit immer die Masse pro Längeneinheit gemeint.

Bei einer vorteilhaften Ausführungsform weist der Mikrowellenresonator zwei gegenphasig schwingende zylindrische koaxiale λ/4-Resonatoren auf, deren Mikrowellenfelder durch auf der Längsachse angeordnete längliche metallische Elemente verstärkt werden, zwischen denen die Materialien senkrecht zur Längsachse hindurchgeführt werden. Auch in diesem Falle hat man wieder ein sehr homogenes Meßfeld.

Bei einer besonders vorteilhaften Ausführungsform ist vorgesehen, daß der Mikrowellenresonator ein zylindrischer koaxialer λ/4-Resonator mit einem auf der Längsachse angeordneten länglichen metallischen Element ist, der an seiner Stirnseite mit maximaler elektrischer Mikrowellenfeldstärke eine im wesentlichen halbkreisförmige Öffnung und daneben einen nach außen offenen schlitzförmigen Durchlaß für die Materialien aufweist, der an das metallische Element angrenzt, während der Rest der Stirnseite geschlossen ist.

Auch bei dieser Ausführungsform hat der Mikrowellenresonator einen von außen zugänglichen Schlitz, in dem das Mikrowellenfeld sehr homogen ist und in das auch ein Endlosfaden problemlos von der Seite her eingeführt werden kann.

Durch die Erfindung wird also eine Vorrichtung geschaffen, mit der die Qualität, insbesondere die Masse pro Länge von strangförmigen Materialien in Form von Garnen, Vorgarnen, Faserbändern und dergleichen genau bestimmt werden kann. Insbesondere ist dabei die Vorrichtung als Laborplatz ausgebildet. Die Vorrichtung ist dabei zur Auswertung der Daten vorzugsweise so ausgebildet, daß sie an einen PC angeschlossen werden kann.

Die Erfindung wird im folgenden anhand von vorteilhaften Ausführungsformen unter Bezugnahme auf die beigefügten Zeichnungen beschrieben. Es zeigen in schematischer Darstellung:
- Fig. 1: den prinzipiellen Aufbau der erfindungsgemäßen Vorrichtung;
- Fig. 2: einen erfindungsgemäßen Resonator;
- Fig. 3: die Mikrowellenfeldstärke als Funktion des Ortes im Resonator des Fig. 2;
- Fig. 4: in perspektivischer Ansicht eine weitere Ausführungsform eines erfindugnsgemäßen Resonators;
- Fig. 5: im Längsschnitt den Resonator der Fig. 4;
- Fig. 6: der Verlauf der elektrischen Feldstärke des Mikrowellenfeldes im Resonator der Fig. 4 und 5;
- Fig. 7 und 8: grafische Darstellungen der Mikrowellenfeldstärke als Funktion des Ortes des Resonators der Fig. 4 bis 6;
- Fig. 9: eine Anordnung zur Messung von verschiedenen Größen von strangförmigen Materialien;
- Fig. 10: eine andere Anordnung zur Messung von verschiedenen Größen strangförmiger Materialien.

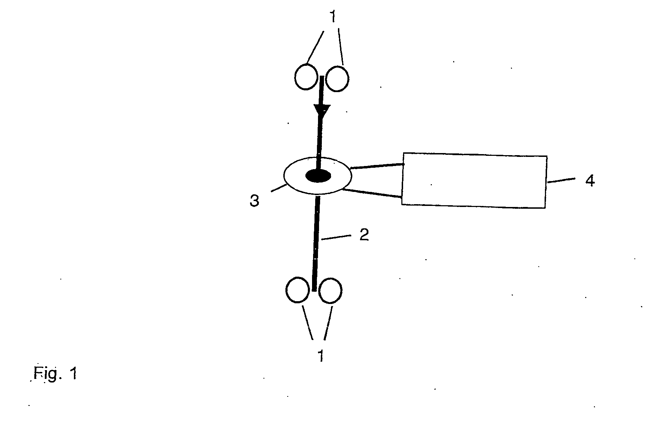

Fig. 1 zeigt den prinzipiellen Aufbau der erfindungsgemäßen Vorrichtung. Über Strangführungen, die bei 1 dargestellt sind, wird das strangförmige Material 2 durch einen Mikrowellenresonator 3 geführt, der mit einer Meßelektronik 4 verbunden ist. Durch die Meßelektronik werden Mikrowellen in den Hohlraum 3 eingestrahlt. Die aus dem Hohlraum ausgekoppelte Mikrowellenstrahlung wird detektiert. Es wird dabei sowohl die Verschiebung der Resonanzfrequenz als auch die Verbreiterung der Resonanzkurve durch das hindurchgeführte strangförmige Material 2 bestimmt.

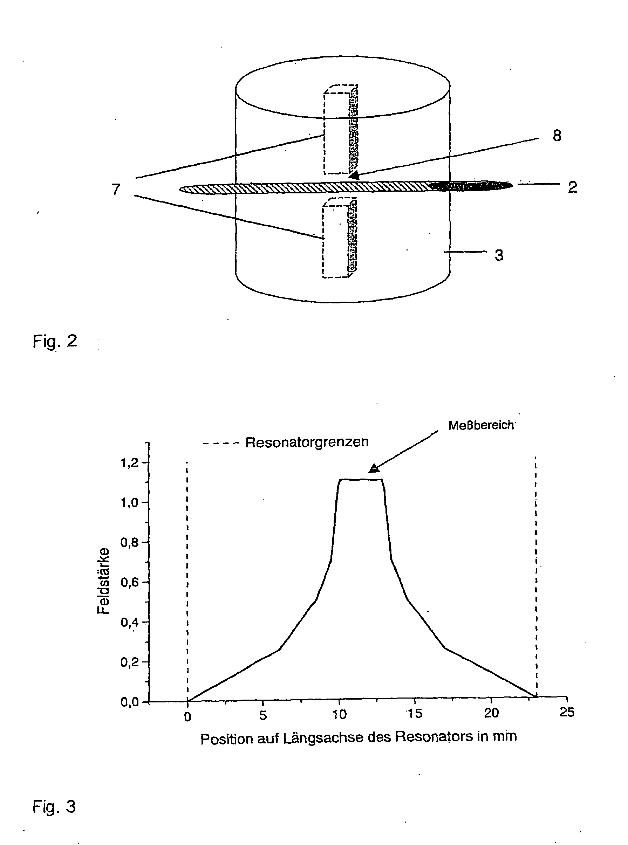

Bei der ersten Ausführungsform der Erfindung der Fig. 2 besteht der Resonator aus zwei gegenphasig schwingenden koaxialen λ/4-Resonatoren. Dieser Resonator ist empfindlich genug, um selbst die Masse dünner Garne zu messen. Die beruht auf der Konzentration des Feldes in einem Raumbereich 8, durch den das Meßgut 2 geführt wird. Diese Konzentration wird mit Hilfe metallischer länglicher Elemente 7 erreicht. Der Feldverlauf dieses Resonators ist in Fig. 3 gezeigt.

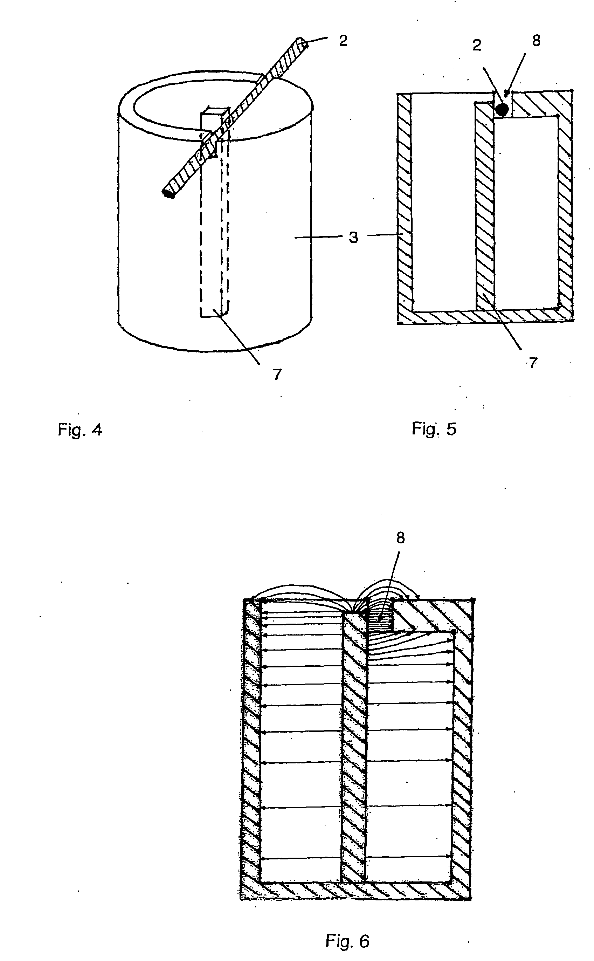

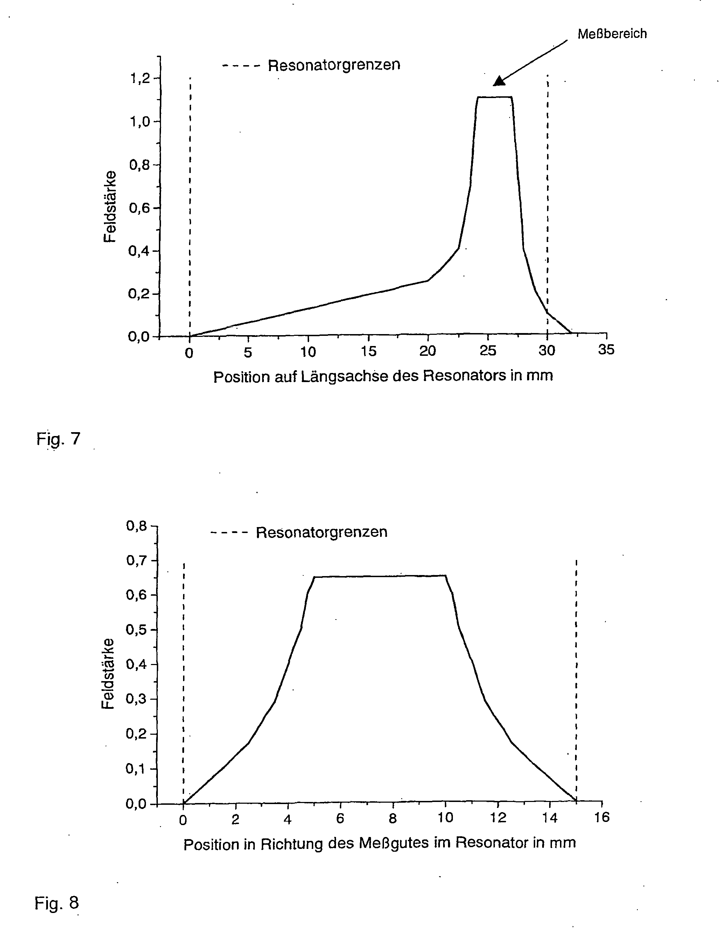

In den Figuren 4 bis 6 ist ein weiterer Resonator der Erfindung gezeigt, der den Vorteil hat, daß der Materialstrang 2 ein Endlosstrang sein kann, der in einen Meßschlitz eingeführt wird. Der Resonator ist ein koaxialer λ/4-Resonator. Dies bedeutet, daß die Feldstärke am Boden des Resonators null ist und zum oberen Rand des Resonators kontinuierlich zunimmt. Die Höhe des Resonators entspricht einem Viertel der Wellenlänge. Auch mit diesem Resonator kann die Masse dünner Garne gemessen werden, was an der Konzentration des Feldes im Raumbereich 8 liegt, durch den dann das Meßgut geführt wird. Die Feldkonzentration wird mit Hilfe eines stabförmigen metallischen Elementes 7, das sich auf der Längsachse des Resonators befindet, im Zusammenwirken mit der metallischen Stirnfläche bewirkt. Der Resonator ist im wesentlichen zylindrisch, wobei die Stirnfläche, bei der die Mikrowellenfeldstärke ein Maximum hat, im wesentlichen halb metallisch offen und halb metallisch geschlossen ist. Der Schlitz zum Aufnehmen des strangförmigen Materials 2 befindet sich dabei leicht exzentrisch neben dem stabförmigen Element 7. Der Verlauf der elektrischen Feldlinien ist in Figur 6 dargestellt, wobei eine höhere Dichte von Feldlinien eine höhere elektrische Feldstärke bedeutet. In den Figuren 7 und 8 ist die Stärke des Mikrowellenfeldes in Richtung der Längsachse des Resonators und in Richtung des Meßgutes 2 aufgetragen.

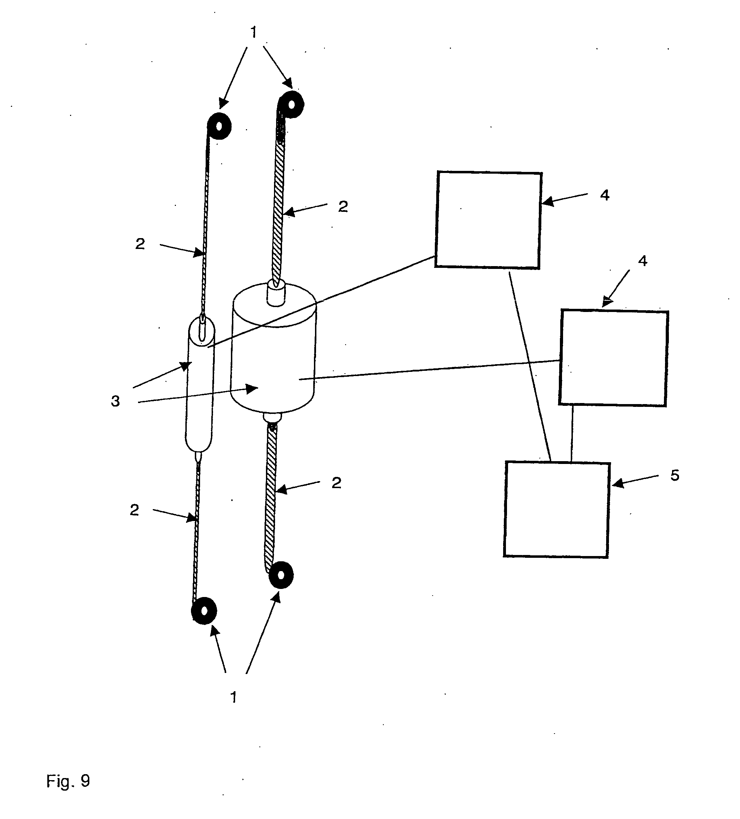

Bei der Ausführungsform der Fig. 9 sind gleichzeitig mehrere, hier nur schematisch dargestellte Resonatoren 3 vorgesehen, die für dünnes Material (links) und dickes Material (rechts) vorgesehen sind. Jeder dieser Mikrowellenresonatoren ist mit einer eigenen Mikrowellenelektronik verbunden, wobei der kleinere Resonator mit höheren Mikrowellenfrequenzen betrieben wird als der größere Resonator. Die Bauform der Resonatoren kann in diesem Falle für alle Resonatoren die gleiche sein. Die Größen der Resonatoren hängen aber von der Arbeitsfrequenz ab. Im höheren Frequenzbereich werden kleinere Resonatoren betrieben, die in der Lage sind, Meßgut mit sehr geringer Maße zu messen. Für dickere Faserbänder dagegen kommen größere Resonatoren bei niedrigerer Frequenz zum Einsatz.

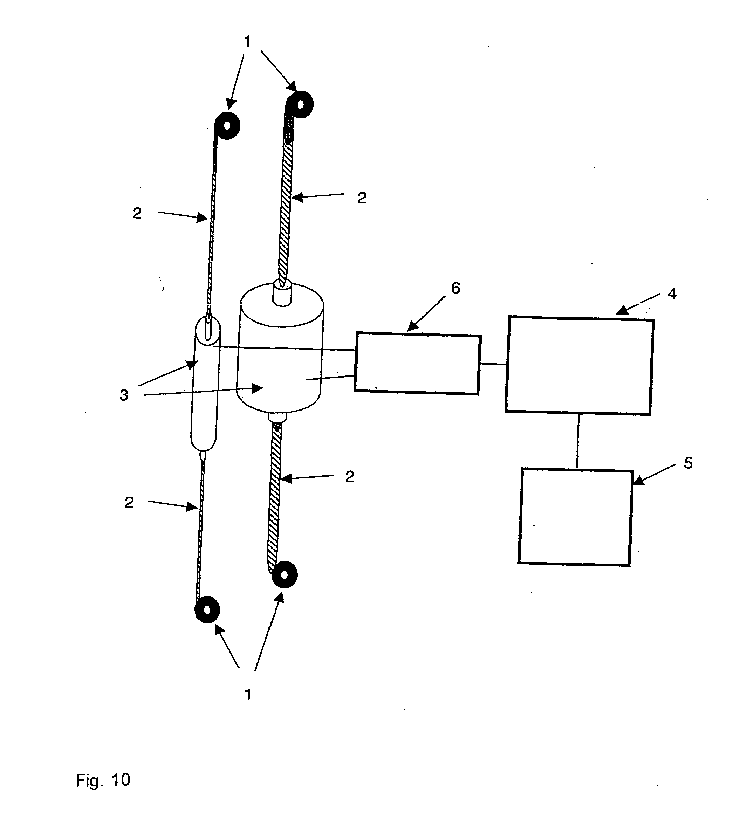

Dies ist auch der Fall bei der Ausführungsform der Fig. 10, wo vorteilhafterweise nur eine Mikrowellenelektronik 4 vorgesehen ist, die jeweils mit einem Umschalter 6 mit dem Mikrowellenresonator 3 verbunden wird, durch den gerade strangförmiges Material 2 hindurchgeführt wird. In diesem Falle ist nur eine Mikrowellen-Elektronik in Kombination mit einem Umschalter nötig, der die verschiedenen Resonatoren ansteuert. Bei Messungen in niedrigem Bereich von 2 bis 3 GHz können für die Messung massereicherer Bänder zylindrische Resonatoren verwendet werden. Für sehr dünne Garne muß trotz der relativ großen Wellenlänge das Feld im Resonator auf kleinstem Raum konzentriert werden, um eine genügende Empfindlichkeit der Resonatoren zu gewährleisten.

Um eine genaue Messung zu ermöglichen, sollte das Mikrowellenfeld des Resonators 3 nicht nur für den Fall dünner Garne im wesentlichen auf das strangförmige Material 2 konzentriert werden. Dies geschieht bei der Ausführungsform der Figur 9 durch verschieden große Resonatoren mit sehr unterschiedlichen Resonanzfrequenzen. Bei der Ausführungsform der Figur 10 werden alle Bandmassen in demselben Frequenzbereich gemessen. Dies geschieht durch Feldkonzentration mit Hilfe interner Strukturen der Resonatoren. Es ist auch möglich, mehrere gleiche Resonatoren, die mit gleichen Frequenzen betrieben werden, nebeneinander anzuordnen, um mehrere Stränge gleichzeitig zu messen.

## Patentansprüche

1. Vorrichtung zur Qualitätsprüfung von strangförmigen Materialien (2) in Form von Garnen, Vorgarnen, Faserbändern und dergleichen mit einer Messeinrichtung, die einen Mikrowellengenerator, einen Mikrowellenresonator (3) und Einrichtungen (4) zum Bestimmen der Verschiebung der Resonanzfrequenz und der Verbreiterung der Resonanzkurve durch das strangförmige Material (2) aufweist, und mit mechanischen Einrichtungen (1) zum Transport der strangförmigen Materialien (2) durch das Messvolumen des Mikrowellenresonators, wobei das Mikrowellenfeld im Messvolumen homogen ist und die Vorrichtung zum Messen der Masse pro Länge und der Feuchte der strangförmigen Materialien (2) ausgebildet ist und **dadurch gekennzeichnet dass** der Mikrowellenresonator (3) ein oder mehrere auf seiner Längsachse angeordnete längliche metallische Elemente (7) zur Konzentration des Mikrowellenfeldes aufweist.

2. Vorrichtung nach Anspruch 1, **dadurch gekennzeichnet, dass** der Mikrowellenresonator (3) zwei gegenphasig, schwingende zylindrische koaxiale λ/4-Resonatoren aufweist, deren Mikrowellenfelder durch zwei auf der Längsachse angeordnete längliche metallische Elemente (7) verstärkt wird, zwischen denen die Materialien senkrecht zur Längsachse hindurchgeführt werden.

3. Vorrichtung nach Anspruch 1, **dadurch gekennzeichnet, daß** der Mikrowellenresonator (3) ein zylindrischer koaxialer λ/4-Resonator mit einem auf der Längsachse angeordneten länglichen metallischen Element (7) ist und der an seiner Stirnseite mit maximaler elektrischer Mikrowellenfeldstärke eine im wesentlichen halbkreisförmige Öffnung, und daneben einen nach außen offenen schlitzförmigen Durchlass (8) für die Materialien (2), der an das metallische Element (7) angrenzt, aufweist, während der Rest der Stirnseite geschlossen ist.

4. Vorrichtung nach einem der Ansprüche 1 bis 3, **dadurch gekennzeichnet; dass** sie mit Frequenzen von 300 MHz bis 30 GHz, insbesondere 1 GHz bis 15 GHz betreibbar ist.

5. Vorrichtung nach einem der Ansprüche 1 bis 4, **dadurch gekennzeichnet, dass** sie als Labormessplatz ausgebildet ist.

6. Vorrichtung nach einem der Ansprüche 1 bis 5, **dadurch gekennzeichnet, dass** sie mehrere Resonatoren (3) aufweist, die über Umschalter (6) mit der Auswertungsschaltung (4) verbindbar sind.

## Claims

1. Device for testing the quality of strand-like materials (2) in the form of yarns, rovings, fibre bands and the like by means of a measuring device which has a microwave generator, a microwave resonator (3) and devices (4) for determining the displacement of the resonant frequency and the widening of the resonance curve due to the strand-like material (2), and having mechanical devices (1) for transporting the strand-like materials (2) through the measuring volume of the microwave resonator, the microwave field being homogeneous in the measuring volume and the device being constructed for measuring the mass per length and the moisture of the strand-like materials (2), and **characterized in that** the microwave resonator (3) has one or more elongated metallic elements (7), arranged on its longitudinal axis, for concentrating the microwave field.

2. Device according to Claim 1, **characterized in that** the microwave resonator (3) has two cylindrical coaxial λ/4 resonators oscillating in opposite phases, the microwave fields of which are amplified by two elongated metallic elements (7) arranged on the longitudinal axis, between which the materials are passed through perpendicularly to the longitudinal axis.

3. Device according to Claim 1, **characterized in that** the microwave resonator (3) is a cylindrical coaxial λ/4 resonator with an elongated metallic element (7) arranged on the longitudinal axis and which has at its front end, with maximum electrical microwave field strength, an essentially semicircular opening and next to it a slot-shaped passage (8) for the materials (2), which is open towards the outside and which adjoins the metallic element (7) whereas the rest of the front end is closed.

4. Device according to one of Claims 1 to 3, **characterized in that** it can be operated at frequencies of 300 MHz to 30 GHz, particularly 1 GHz to 15 GHz.

5. Device according to one of Claims 1 to 4, **characterized in that** it is constructed as a laboratory test station.

6. Device according to one of Claims 1 to 5, **characterized in that** it has a number of resonators (3) which can be connected to the evaluating circuit (4) via change-over switches (6).

## Revendications

1. Dispositif pour tester la qualité de matériaux en cordon (2), en forme de fils, de mèches, de bandes de fibres et analogue, au moyen d'un dispositif de mesure comportant un générateur de micro-ondes, un résonnateur à micro-ondes (3) et des dispositifs (4) pour déterminer la dérive de la fréquence de résonnance et de la propagation de la courbe de résonnance à travers les matériaux en cordon (2) et comportant des dispositifs mécaniques (1) pour le transport des matériaux en cordon (2) à travers le volume de mesure du résonnateur à micro-ondes, le champ de micro-ondes étant homogène dans le volume de mesure et le dispositif étant réalisé pour mesurer la masse linéaire et l'humidité des matériaux en cordon (2), **caractérisé en ce que** le résonnateur à micro-ondes (3) présente sur son axe longitudinal un ou plusieurs éléments métalliques allongés (7) pour concentrer le champ de micro-ondes.

2. Dispositif selon la revendication 1, **caractérisé en ce que** le résonnateur à micro-ondes (3) présente deux résonnateurs λ/4 cylindriques coaxiaux oscillants, de phase opposée, dont les champs de micro-ondes sont amplifiés par deux éléments métalliques allongés (7) disposés sur l'axe longitudinal, entre lesquels les matériaux sont guidés perpendiculairement à l'axe longitudinal.

3. Dispositif selon la revendication 1, **caractérisé en ce que** le résonnateur à micro-ondes (3) est un résonnateur λ/4 cylindrique coaxial avec un élément métallique allongé (7) disposé sur l'axe longitudinal et qui présente, sur sa face frontale avec une intensité de champ électrique de micro-ondes, une ouverture essentiellement semi-circulaire et, à côté, un passage (8) en forme de fente ouvert vers l'extérieur pour les matériaux en cordon (2), qui avoisine l'élément métallique tandis que le reste de la face frontale est fermé.

4. Dispositif selon l'une des revendications 1 à 3, **caractérisé en ce qu'**il fonctionne avec des fréquences de 300 MHz à 30 GHz, en particulier de 1 GHZ à 15 GHz.

5. Dispositif selon l'une des revendications 1 à 4, **caractérisé en ce qu'**il est conformé en poste de mesure de laboratoire.

6. Dispositif selon l'une des revendications 1 à 5, **caractérisé en ce qu'**il présente plusieurs résonnateur (3) qui peuvent être reliés par un commutateur (6) au circuit d'esploitation (4).
